# EUROPEAN PATENT SPECIFICATION

(11) **EP 2 552 445 B1**
(45) Date of publication and mention of the grant of the patent: **24.01.2018**
(21) Application number: 11720347.1
(22) Date of filing: 31.03.2011
(51) Int. Cl.: A61K 31/485, A61K 9/00, A61K 9/06, A61K 9/48, A61K 47/32, A61K 47/44, A61P 31/22

(54) **OPIOID RECEPTOR ANTAGONIST (NALTREXONE) FOR USE IN TREATING HERPES ZOSTER DISEASE**
OPIOID REZEPTOR ANTAGONIST (NALTREXON) ZUR VEWENDUNG IN DER BEHANDLUNG VON HERPES ZOSTER ERKRANKUNGEN
ANTAGONISTS DU RECEPTEUR OPIOID (NALTREXONE) POUR L'UTILISATION DANS LE TRAITEMENT DES MALADIES DU HERPES ZOSTER

(30) Priority: 31.03.2010 TR 201002473
(43) Date of publication of application: 06.02.2013
(73) Proprietor: Imuneks Farma Ilaç Sanayi Ve Ticaret A.S., 34349 Istanbul (TR)
(72) Inventor: PISAK, Ibrahim Mustafa Iskender, Gayrettepe / Istanbul 34349 (TR); PISAK, Mehmet, Gayrettepe / Istanbul 34349 (TR); SELAMOGLU, Mehmet Levent, Gayrettepe / Istanbul 34349 (TR); BINGOL, Semra, Gayrettepe / Istanbul 34349 (TR)
(74) Representative: Coral, Nükhet Serra Yardimci
(86) International application number: PCT/TR2011/000074
(87) International publication number: WO 2011/123084

(56) References cited:
- EP-A1- 1 813 283
- WO-A2-2004/082588
- US-A- 4 888 346
- US-A- 5 356 900

## Description

### Field of the Invention

The present invention relates to the new topical, oral or ophthalmic pharmaceutical compositions comprising opioid antagonists, such as naltrexone, naloxone, or nalmefene, or pharmaceutically acceptable salts thereof, and the use of said pharmaceutical compositions for treating conditions caused by the varicella-zoster virus, such as herpes zoster disease.

### Background of the Invention

Naltrexone is a pure opioid antagonist with the chemical name of morphinan-6-one, 17-(cyclopropylmethyl)-4,5-epoxy-3,14-dihydroxy-(5α). The molecular formula of naltrexone is C₂₀H₂₃NO₄ and its molecular weight is 341.41 in the anhydrous form (< 1% maximum water content). The chemical structure of naltrexone is shown below.

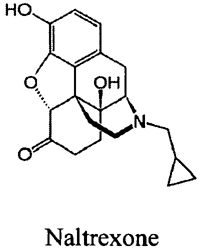

Naltrexone has been approved in 1980s for use in the treatment of alcoholism or narcotic addiction. It is believed that naltrexone functions by blocking the brain receptors that trigger the effects of alcohol or narcotics. It is usually available in the market as tablets of 50 mg suitable for once daily administration. A new formulation of extended-release suspension for intramuscular injection has been approved by FDA in 2006.

Naloxone is the first synthesized pure opioid antagonist with the chemical name of (-)-17-allyl-4,5α-epoxy-3,14-dihydroxymorphinan-6-one and the molecular formula of C₁₉H₂₁NO₄. The chemical structure of naloxone is shown below.

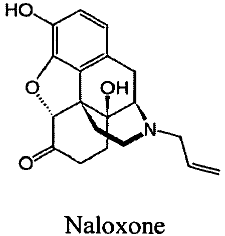

Naloxone is typically administered intravenously because of its short duration of action and low oral bioavailability, and is generally administered to a patient in order to reverse opioid depression, including respiratory depression, induced by natural and synthetic opioids.

Nalmefene has the chemical name of 17-cyclopropylmethyl-4,5α-epoxy-6-methylenemorphinan-3,14-diol and the molecular formula of C₂₁H₂₅NO₃. The chemical structure of nalmefene is shown below.

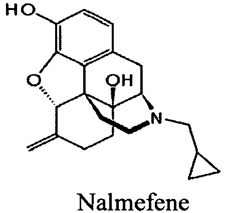

Nalmefene is typically used in the management of alcohol dependence, and also has been investigated for the treatment of other addictions such as pathological gambling and addiction to shopping. Nalmefene is an opiate derivative similar in both structure and activity to the opiate antagonist naltrexone. Advantages of nalmefene relative to naltrexone include longer half-life, greater oral bioavailability and no observed dose-dependent liver toxicity. Like other drugs of this type, nalmefene can precipitate acute withdrawal symptoms in patients who are dependent on opioid drugs, or more rarely when used post-operatively to counteract the effects of strong opioids used in surgery.

However in the early 1980s, professor Ian Zagon discovered that low dose naltrexone (LDN) increases the production of endogenous endomorphins and increases the density of opiate receptors. Especially low dose naltrexone increases production of the endogenous pentapeptide metenkephalin, also named by Zagon *opioid growth factor* (OGF), as well as the number and density of OGF receptors by intermittently blocking the opiate receptors. This intermittent increase enhances homostatic regulation of the natural immune function of the human body. Zagon's publications inspired Bernard Bihari and his team who began studying low dose naltrexone in patients with AIDS and described the results in the US patent 4,888,386. They applied these principles to study the use of low dose naltrexone in patients affected by different types of immune-mediated conditions like multiple sclerosis, lymphoma, cancer, etc. In particular, they studied the oral administration of low dose naltrexone in patients suffering from chronic herpes infection, especially genital herpes and described the results in the US patent 5,356,900. In view of Zagon's findings, Bernard Bihari reported the use of low dose naltrexone for the treatment of patients with AIDS (U.S. Patent No. 4,888,346) and herpes (U.S. Patent No. 5,356,900).

But Bihari et al. limited in U.S. Patent No. 5,356,900 the invention to particularly genital herpes and multiple sclerosis. Bihari et al. could not find a clear connection between chronic infections caused by herpes virus, both herpes simplex virus and Epstein-Barr-virus, such as genital herpes and mononucleosis, and multiple sclerosis with varicella-zoster virus-associated conditions. Even infections caused by herpes virus and varicella-zoster virus-associated conditions are clinically distinct diseases with different symptoms and modes of transmission.

In U.S. Patent No. 4,888,346 Bihari et al. does not mention varicella-zoster virus or varicella-zoster virus-associated conditions, and does not teach a method of treating any condition/disease caused by varicella-zoster virus. Bihari et al. only concentrate on a method treating HTLV-III virus infections which are not associated with varicella-zoster virus-associated infections, and which are also clinically distinct diseases with different symptoms and modes of transmission. The results of this disclosure do not motivate to use an opioid antagonist in the treatment of varicella-zoster virus-associated conditions as well.

But also other inventors have been working with opioid antagonists.

Tanabe et al. presented in E.P. Patent no. 1813283 the use of an opioid antagonist selected from a large list for neuropathic pain that may result from a large group of diseases including postherpetic neuralgia. This invention is not focused on the treatment of varicella-zoster virus-associated conditions itself.

Britten et al. teachs in WO2004082588 a different method of treating an inflammatory condition that may be associated with a large group of diseases including herpes zoster oticus and geniculate herpes using an anti-inflammatory agent e.g. levallorphan, nalorphine etc., selected from a large list other than opioid antagonists. But levallorphan and nalorphine are analgesics which are not included in the same therapeutic class such as opioid antagonists, preferably nalmefene, naloxone and naltrexone, so they have different mechanism of action and different therapeutic effects.

Nicholas Plotnikoff studied the antiviral properties of the OGF whose production is increased by administration of LDN and reported that LDN can be an effective therapy against herpes, HIV infection, cytomegalovirus, coronavirus, influenza A and Japanese encephalitis (The Promise of Low Dose Naltrexone Therapy, Elaine A. Moore and Samantha Wilkinson, McFarland & Company Inc Publishers, 2009).

Further, Nicholas Plotnikoff reported the use of low dose naltrexone for the treatment of herpes, HIV infection, cytomegalovirus, coronavirus, influenza A and Japanese encephalitis (E.A. Moore & S. Wilkinson, THE PROMISE OF Low DOSE NALTREXONE THERAPY: POTENTIAL BENEFITS IN CANCER, AUTOIMMUNE, NEUROLOGICAL AND INFECTIOUS DISORDERS (McFarland & Company, Inc., Publishers, 2009)).

Herpes zoster (or shingles) is a disease caused by the reactivation of the varicella-zoster virus (VZV). The VZV causes chickenpox (or varicella) generally during childhood and lies in a dormant state in nerve cells along the spine. It can re-emerge as shingles in adults and is more common in people over 60 or those with a weak immune system. The main symptom of shingles is a unilateral vesicular eruption with dermatomal distribution. The onset of the disease is preceded by pain 48 to 72 hours before the rash develops in the affected dermatome. The rash appears as erythematous, maculopapular lesions that rapidly evolve into vesicles. The vesicles may coalesce and form bullous lesions. The lesions continue to form for 3 to 5 days, with a total duration of the disease of 10 to 15 days. However it can take as long as 1 month before the skin returns to normal.

Herpes zoster can also affect the surrounding regions of the eye and the eye itself. This is called herpes zoster ophthalmicus - the reactivation of a varicella-zoster virus infection involving the eye. Symptoms and signs, which may be intense, include dermatomal forehead rash and painful inflammation of all the tissues of the anterior and, rarely, posterior structures of the eye. Complications may include: keratitis accompanied by uveitis; late sequelae, such as glaucoma, cataract, chronic or recurrent uveitis, corneal scarring, corneal neovascularization, or hypesthesia; and postherpetic neuralgia (Herpes zoster ophthalmicus, The Merck manual of diagnosis and therapy, The Merck manuals online medical library. http://www.merck.com/mmpe/sec09/ch102/ch102e.html#sec09-ch102-ch102e-355, Accessed January 6, 2010).

Current treatments for herpes zoster include topical, oral, or ophthalmic administration of an antiviral agent, such as acyclovir.

By using the antiviral properties of metenkephalin produced after application of LDN, the present invention can offer a new safe and simple alternative for the treatment of the disease of herpes zoster.

### Summary of the Invention

In one embodiment, the invention relates to the use of an opioid antagonist in the manufacture of a medicament for the treatment of a condition caused by reactivation of the varicella-zoster virus comprising administering to a subject in need thereof an effective amount of an opioid antagonist or a pharmaceutically acceptable salt thereof.

In another embodiment, the invention also relates to the use of an opioid antagonist in the manufacture of a kit for the treatment of a condition caused by varicella-zoster virus, wherein the kit comprises a unit dose of an opioid antagonist and a label or printed instructions instructing the administration of the opioid antagonist to treat a condition caused by varicella-zoster virus.

### Detailed Description of the Invention

The present invention relates to the use for treating conditions caused by the varicella-zoster virus comprising administering to a subject in need thereof an effective amount of an opioid antagonist.

As used herein, an "effective amount" is an amount effective for treating conditions caused by the varicella-zoster virus.

As used herein, the term "treating" conditions caused by the varicella-zoster virus in a subject refers to subjecting the subject to a pharmaceutical treatment, e.g., the administration of one or more agents, such that at least one symptom of the condition is decreased or prevented from worsening.

Conditions caused by the varicella-zoster virus include, but are not limited to, herpes zoster disease (also called shingles). Conditions associated with herpes zoster disease include, but are not limited to, shingles vesicular rash, postherpetic neuralgia, zoster multiplex, zoster sine herpete, myelitis, Ramsay-Hunt syndrome, and herpes zoster ophthalmicus.

Suitable opioid antagonists include compounds that block one or more opioid receptors. In some embodiments, the opioid antagonist selectively blocks the mu (µ) opioid receptor, the delta (δ) opioid receptor, or the kappa (κ) opioid receptor. In other embodiments, the opioid antagonist is non-selective. Examples of opioid antagonists include, but are not limited to, naltrexone, naloxone, nalorphine, levallorphan, nalmefene, cyprodime, naltindole, and norbinaltorphimine. In one embodiment, the opioid antagonist is naltrexone. In another embodiment, the opioid antagonist is naloxone. In another embodiment, the opioid antagonist is nalmefene.

In some embodiments, the opioid antagonist is a compound that may exist in the form of one or more stereoisomers, wherein one or more of those stereoisomers is therapeutically active. In some embodiments, the opioid antagonist comprises a therapeutically active stereoisomer that is substantially free of other stereoisomers. In other embodiments, the opioid antagonist comprises a therapeutically active stereoisomer that has less than about 50%, less than about 40%, less than about 30%, less than about 20%, less than about 10%, less than about 5%, or less than about 1% by weight of other stereoisomers.

The opioid antagonists of the invention are typically administered to the subject in the form of a composition for topical, oral, or ophthalmic administration. The total daily dose of the opioid antagonists of the invention is preferably up to 150 mg/cm² of skin topically, preferably up to 10 mg orally and preferably from 0.1% to 10% by weight ophthalmically.

The pharmaceutical compositions of the invention comprise an effective amount of the opioid antagonist and at least one pharmaceutically acceptable excipient.

The use of the pharmaceutical compositions of the invention for the treatment of herpes zoster disease caused by reactivation of the varicella-zoster virus (VZV) results in the complete healing of the herpes zoster affected area.

### Compositions for topical administration

In some embodiments, the opioid antagonist is administered to the subject in the form of a composition for topical administration.

In some embodiments, the opioid antagonist is present in the topical compositions of the invention in an amount of from about 0.1% to about 5% by weight of the composition. In other embodiments, the opioid antagonist is present in an amount of from about 0.5% to about 4%, from about 0.5% to about 3%, from about 0.5% to about 2%, from about 0.5% to about 1%, from about 1% to about 4%, from about 1% to about 3%, from about 1% to about 2%, from about 2% to about 4%, from about 2% to about 3%, from about 3% to about 4%, or about 1% by weight of the composition.

Suitable pharmaceutically acceptable excipient for the topical compositions of the invention may be any topically acceptable non-transdermally effective excipient known by those skilled in the art. Suitable excipients include, but are not limited to, emulsifying agents, stiffening agents, rheology modifiers or thickeners, surfactants, emollients, preservatives, humectants, alkalizing or buffering agents, and solvents.

Suitable emulsifying agents include, but are not limited to, cetyl alcohol, cetostearyl alcohol, stearyl alcohol, carboxypolymethylene, polycarbophil, polyethylene glycol, and sorbitan esters. Suitable stiffening agents include, but are not limited to, stearyl alcohol, cetostearyl alcohol, and cetyl alcohol. Suitable rheology modifiers or thickeners include, but are not limited to, carbomers such as Carbopol®, and polyoxyethylene tallow amines such as Ethomeen®. Suitable surfactants include, but are not limited to, anionic, cationic, amphoteric, and nonionic surfactants, such as sodium lauryl sulfate, cetostearyl alcohol, cetyl alcohol, magnesium lauryl sulfate, a wax, or a combination thereof. Suitable emollients include, but are not limited to, white petrolatum (while vaseline), liquid petrolatum (liquid vaseline), paraffin, or aquaphor. Suitable preservatives include, but are not limited to, antimicrobial preservatives such as nipagin (methyl hydroxybenzoate), nipasol (hydroxybenzoate), butylparaben, ethylparaben, methylparaben, propyl paraben potassium, and propyl paraben sodium. Suitable humectants include, but are not limited to, propylene glycol and propylene glycol alginate. Suitable alkalizing or buffering agents include, but are not limited to, sodium hydroxide and potassium hydroxide. Suitable solvents include, but are not limited to, water.

The topical compositions of the invention may be in the form of a gel, cream, ointment, liquid, suspension, solution, emulsion, foam, aerosol or the like for topical administration. Typically, the composition is administered to the subject by spreading (*e.g*., gel, cream, or ointment) or spraying (*e.g*., liquid) onto the affected area of the skin.

In one embodiment, the composition is in the form of cream. Typically, the cream compositions of the invention comprise an opioid antagonist and one or more of an emulsifying agent, a stiffening agent, a surfactant, an emollient, a preservative, a humectant, an alkalizing or buffering agent, and a solvent. The cream has a composition according to Table 1a, 1b, or 1c.

**Table 1a. Illustrative Cream Composition**

| **Ingredient** | **Amount (% by weight of the composition)** |
|---|---|
| Opioid antagonist | about 0.1% - 5% |
| Emulsifying agent | about 2% - 5% |
| Stiffening agent | about 1% - 45% |
| Surfactant | about 0.5% - 2.5% |
| Preservative | about 0.01 % - 0.6% |
| Humectant | about 1% - 15% |
| Alkalizing or buffering agent | about 0.01% - 3% |
| Emollient | about 1% - 50% |
| Solvent | q.s. |

**Table 1b. Illustrative Cream Composition**

| **Ingredient** | **Amount (% by weight of the composition)** |
|---|---|
| Opioid antagonist | about 0.5% - 2% |
| Emulsifying agent | about 2% - 5% |
| Stiffening agent | about 2% - 5% |
| Surfactant | about 0.5% - 1.5% |
| Preservative | about 0.01% - 0.6% |
| Humectant | about 2% - 10% |
| Alkalizing or buffering agent | about 0.01% - 3% |
| Emollient | about 15% - 30% |
| Solvent | q.s. |

**Table 1c. Illustrative Cream Composition**

| **Ingredient** | **Amount (% by weight of the composition)** |
|---|---|
| Opioid antagonist | about 0.5% - 2% |
| Cetyl alcohol and/or carboxypolymethylene | about 2% - 5% |
| Stearyl alcohol | about 2% - 5% |
| Sodium lauryl sulfate | about 0.5% - 1.5% |
| Nipagin and/or Nipasol | about 0.01% - 0.6% |
| Propylene glycol | about 2% - 10% |
| Sodium hydroxide | about 0.01% - 3% |
| White vaseline and/or liquid vaseline | about 15% - 30% |
| Water | q.s. |

In one embodiment, the composition is in the form of ointment. Typically, the ointment compositions of the invention comprise an opioid antagonist and one or more of an emulsifying agent, an emollient, and a preservative. The ointment has a composition according to Table 2a, 2b, or 2c.

**Table 2a. Illustrative Ointment Composition**

| **Ingredient** | **Amount (% by weight of the composition)** |
|---|---|
| Opioid antagonist | about 0.1% - 5% |
| Emulsifying agent | about 1% - 10% |
| Preservative | about 0.01% - 0.6% |
| Emollient | q.s. |

**Table 2b. Illustrative Ointment Composition**

| **Ingredient** | **Amount (% by weight of the composition)** |
|---|---|
| Opioid antagonist | about 0.5% - 2% |
| Emulsifying agent | about 2% - 5% |
| Preservative | about 0.01% - 0.6% |
| Emollient | q.s. |

**Table 2c. Illustrative Ointment Composition**

| **Ingredient** | **Amount (% by weight of the composition)** |
|---|---|
| Opioid antagonist | about 0.5% - 2% |
| Polyoxyethylene 20 sorbitan monooleate | about 2% - 5% |
| Nipagin and/or Nipasol | about 0.01% - 0.6% |
| White vaseline and/or liquid vaseline | q.s. |

In one embodiment, the composition is in the form of gel. Typically, the gel compositions of the invention comprise an opioid antagonist and one or more of a rheology modifier or thickener, an alkalizing or buffering agent, and a solvent. The gel has a composition according to Table 3a, 3b, or 3c.

**Table 3a. Illustrative Gel Composition**

| **Ingredient** | **Amount (% by weight of the composition)** |
|---|---|
| Opioid antagonist | about 0.1% - 5% |
| Rheology modifier or thickener | about 0.5% - 2% |
| Alkalizing or buffering agent | about 0.5% - 10% |
| Solvent | q.s. |

**Table 3b. Illustrative Gel Composition**

| **Ingredient** | **Amount (% by weight of the composition)** |
|---|---|
| Opioid antagonist | about 0.5% - 2% |
| Rheology modifier or thickener | about 1% - 2% |
| Alkalizing or buffering agent | about 0.5% - 5% |
| Solvent | q.s. |

**Table 3c. Illustrative Gel Composition**

| **Ingredient** | **Amount (% by weight of the composition)** |
|---|---|
| Opioid antagonist | about 0.5% - 2% |
| Carbomer | about 1% - 2% |
| Sodium hydroxide | about 0.5% - 5% |
| Water | q.s. |

Typically, the opioid antagonists of the invention are administered to the subject in a total daily dose of up to about 150 mg/cm² of skin. In some embodiments, the opioid antagonist is administered to the subject in a total daily dose of from about 5 mg/cm² of skin to about 150 mg/cm² of skin, from about 10 mg/cm² of skin to about 100 mg/cm² of skin, from about 20 mg/cm² of skin to about 90 mg/cm² of skin, from about 30 mg/cm² of skin to about 80 mg/cm² of skin, from about 40 mg/cm² of skin to about 70 mg/cm² of skin, or about 50 mg/cm² of skin or about 60 mg/cm² of skin. The total daily dose may be delivered once per day, or divided between multiple doses. In some embodiments, the opioid antagonist is administered 1, 2, 3, 4, or 5 times per day.

### Composition for oral administration

In some embodiments, the opioid antagonist is administered to the subject in the form of a composition for oral administration.

In some embodiments, the opioid antagonist is present in the oral compositions of the invention in an amount of from about 0.1 mg to about 10 mg, from about 0.5 mg to about 8 mg, from about 1 mg to about 6 mg, from about 1 mg to about 4.5 mg, or about 2 mg.

Suitable pharmaceutically acceptable excipients for the solid oral compositions of the invention include, but are not limited to, starches, sugars, diluents, granulating agents, lubricants, binders, disintegrating agents and the like. Suitable pharmaceutically acceptable excipients for the liquid oral compositions of the invention include, but are not limited to, water, glycols, oils, alcohols, flavoring agents, preservatives, coloring agents and the like.

The oral compositions of the invention may be in the form of a tablet, capsule, caplet, granule, powder, lozenge, troche, dragee, sachet, cachet, liquid, solution, suspension, emulsion, elixir, or syrup; preferably in the form of a tablet or capsule. The tablet may be in the form of an uncoated tablet, coated tablet (for example with sugar or an enteric coating), effervescent tablet, dispersible tablet, orally-dissolving tablet, or sublingual tablet.

### Compositions for ophthalmic administration

In some embodiments, the opioid antagonist is administered to the subject in the form of a composition for ophthalmic administration.

In some embodiments, the opioid antagonist is present in the ophthalmic compositions of the invention in an amount of from about 0.1% to about 10% by weight of the composition.

Suitable pharmaceutically acceptable excipients for the ophthalmic compositions of the invention include those known by the skilled artisan to be suitable for ophthalmic administration.

The ophthalmic composition of the invention may be in the form of a solution or a suspension.

The topical, oral, and ophthalmic compositions of the invention may be administered to the subject daily, every other day, three times a week, twice a week, once a week, or at other appropriate intervals. The compositions of the invention are administered until there is complete healing of the condition caused by the varicella-zoster virus in the affected area.

The present invention may use lower doses of opioid antagonist than the doses conventionally used for oral administration in the treatment of alcohol or narcotic addiction. The opioid antagonist is administered to the individual in an amount effective to treat the condition caused by the varicella-zoster virus. The exact dose of opioid antagonist depends upon, by way of non-limiting example, the form in which the opioid antagonist is administered, the subject to be treated, the age, body weight and/or height of the subject to be treated, the preference and experience of the attending physician, the specific opioid antagonist used, the characteristics of the patient, and/or the nature of the condition for which the treatment is sought. Thus, the dosage of opioid antagonist administered may vary from those disclosed herein. These factors are determined by those skilled in the medical and pharmaceutical arts in view of the present disclosure.

The pharmaceutical compositions of the invention may further comprise administration of one or more additional agents effective to treat the condition caused by the varicella-zoster virus. Where the condition is herpes zoster disease, the additional agent may include, but not be limited to, an antiviral agent such as acyclovir, valacyclovir or famciclovir. The additional agent and the opioid antagonist may be administered concurrently or separately. When administered concurrently, the additional agent and the opioid antagonist may be administered in the same or separate compositions.

The present invention also relates to the kits comprising a unit dose of opioid antagonist. The unit dose is within a container, which can be sterile, containing an effective amount of opioid antagonist and a pharmaceutically acceptable excipient. The kits can further comprise a label or printed instructions instructing the use of the opioid antagonist to treat a condition caused by the varicella-zoster virus. The kits can further comprise a device that is useful for administering the unit dose as described herein. Examples of such a device include, but are not limited to, a wand, a dropper, a cotton swab, a pad, or the like.

Having described the invention with reference to certain embodiments, other embodiments will become apparent to one skilled in the art from consideration of the specification. The invention is further defined by reference to the following examples. It will be apparent to those skilled in the art that many modifications, both to materials and methods, may be practiced without departing from the scope of the invention.

### Examples

### Example 1: Treatment of Vesicular Eruption of Herpes Zoster

### Topical composition of naltrexone

100 g of naltrexone 1% cream was prepared by mixing 1 g of naltrexone hydrochloride in 99 g of a cream base containing the following excipients: cetyl alcohol, stearyl alcohol, sodium lauryl sulfate, vaseline, nipagin, nipasol, carboxypolymethylene, propyleneglycol, sodium hydroxide and distilled water.

A 26 year old man with vesicular eruption of herpes zoster on his right forearm and symptoms including hyperesthesia (oversensitivity), itching and pain, was treated with the 1% naltrexone cream described above. The naltrexone cream was administered to the affected area three times daily (morning, noon, and evening) in a total daily amount of 1 g, and the itching, pain and hyperesthesia disappeared after 3 days of treatment. In addition, the vesicular eruption regressed and completely disappeared after 5 days of treatment. No side effects were observed and additional treatment was unnecessary.

### Oral composition of naltrexone

A 53 year old man with a vesicular eruption (rash) of herpes zoster in the form of a belt located on his upper back and symptoms including itching and pain, was treated with the naltrexone capsule. The naltrexone capsule was administered once daily before bedtime in an amount of 2 mg, and the itching and pain disappeared after 4 days of treatment. The rash completely disappeared after 5 days of treatment. No side effects were observed and additional treatment was unnecessary.

### Example 2: Naltrexone Oral and Topical Compositions

The followings are illustrative naltrexone compositions according to the present invention.

**Table 4. Illustrative Naltrexone Capsule Composition**

| **Ingredient** | **Amount (mg)** |
|---|---|
| Naltrexone hydrochloride | 2.00 mg |
| Magnesium stearate | 0.18 mg |
| Microcristalline cellulose | 90.00 mg |
| Gelatin capsule No 4 | 1 |

**Table 5. Illustrative Naltrexone Cream Composition**

| **Ingredient** | **Amount (% by weight of the composition)** |
|---|---|
| Naltrexone | 1% |
| Cetyl alcohol | 3.6% |
| Stearyl alcohol | 3.6% |
| Sodium lauryl sulfate | 0.8% |
| Nipagin | 0.1% |
| Nipasol | 0.05% |
| Carboxylpolymethylene | 0.2% |
| Propylene glycol | 5% |
| Sodium hydroxide | 0.03% |
| White vaseline | 13.5% |
| Liquid vaseline | 5.4% |
| Water | q.s. |

**Table 6. Illustrative Naltrexone Ointment Composition**

| **Ingredient** | **Amount (% by weight of the composition)** |
|---|---|
| Naltrexone | 1% |
| Polyoxyethylene 20 sorbitan monooleate | 4% |
| Nipagin | 0.18% |
| Nipasol | 0.02% |
| White vaseline | 10% |
| Liquid vaseline | q.s. |

**Table 7. Illustrative Naltrexone Gel Composition**

| **Ingredient** | **Amount (% by weight of the composition)** |
|---|---|
| Naltrexone | 1% |
| Carbomer | 2% |
| Sodium hydroxide | 1.25% |
| Water | q.s. |

## Claims

1. The use of an opioid antagonist or a pharmaceutically acceptable salt thereof in the manufacture of a medicament for the treatment of a condition caused by the varicella-zoster virus.

2. The use according to claim 1, wherein the opioid antagonist is naloxone.

3. The use according to claim 1, wherein the opioid antagonist is nalmefene.

4. The use according to claim 1, wherein the opioid antagonist is naltrexone.

5. The use according to any one of claims 1 to 4, wherein the condition caused by the varicella-zoster virus is herpes zoster.

6. The use according to any one of claims 1 to 4, wherein the condition caused by the varicella-zoster virus is herpes zoster ophthalmicus.

7. The use according to any one of claims 1 to 6, wherein the opioid antagonist is administered in the form of a composition comprising the opioid antagonist and at least one pharmaceutically acceptable excipient.

8. The use according to claim 7, wherein the composition is formulated for topical administration.

9. The use according to claim 8, wherein the composition comprises the opioid antagonist in an amount of from 0.1% to 5% by weight of the composition.

10. The use according to claim 8, wherein the composition comprises the opioid antagonist in an amount of 1% by weight of the composition.

11. The use according to any one of claims 8 to 10, wherein the composition is in the form of a gel, cream, ointment, liquid, suspension, solution, emulsion, foam, or aerosol administered to the subject by spreading or spraying the composition onto the affected area.

12. The use according to any one of claims 8 to 11, wherein the opioid antagonist is administered in a total daily dose of up to 150 mg/cm² of skin.

13. The use according to any one of claims 8 to 11, wherein the opioid antagonist is administered in a total daily dose of 50 mg/cm² of skin.

14. The use according to claim 7, wherein the composition is formulated for oral administration.

15. The use according to claim 14, wherein the composition comprises the opioid antagonist in an amount of from 0.1 mg to 10 mg.

16. The use according to claim 14, wherein the composition comprises the opioid antagonist in an amount of 2 mg.

17. The use according to any one of claims 14 to 16, wherein the composition is in the form of a tablet or capsule.

18. The use according to claim 7, wherein the composition is formulated for ophthalmic administration.

19. The use according to claim 18, wherein the composition comprises the opioid antagonist in an amount of from 0.1% to 10% by weight of the composition.

20. The use according to claim 18 or 19, wherein the composition is in the form of a solution or suspension.

21. The use according to any one of claims 1 to 20, wherein the opioid antagonist is administered in 1 to 5 times daily.

22. The use according to any one of claims 1 to 21, wherein the composition further comprises one or more additional agents effective to treat the condition.

23. The use according to claim 22, wherein the additional agent is acyclovir, valacyclovir, or famciclovir.

## Patentansprüche

1. Verwendung eines Opioidantagonisten oder eines pharmazeutisch unbedenklichen Salzes davon in der Herstellung eines Arzneimittels für die Behandlung eines Leidens, das vom Varizella-Zoster-Virus verursacht wird.

2. Verwendung nach Anspruch 1, wobei es sich bei dem Opioidantagonisten um Naloxon handelt.

3. Verwendung nach Anspruch 1, wobei es sich bei dem Opioidantagonisten um Nalmefen handelt.

4. Verwendung nach Anspruch 1, wobei es sich bei dem Opioidantagonisten um Naltrexon handelt.

5. Verwendung nach einem der Ansprüche 1 bis 4, wobei es sich bei dem Leiden, das vom Varizella-Zoster-Virus verursacht wird, um Herpes Zoster handelt.

6. Verwendung nach einem der Ansprüche 1 bis 4, wobei es sich bei dem Leiden, das vom Varizella-Zoster-Virus verursacht wird, um Herpes Zoster Ophthalmicus handelt.

7. Verwendung nach einem der Ansprüche 1 bis 6, wobei der Opioidantagonist in Form einer Zusammensetzung, die den Opioidantagonisten und mindestens einen pharmazeutisch unbedenklichen Grundstoff umfasst, verabreicht wird.

8. Verwendung nach Anspruch 7, wobei die Zusammensetzung für die topische Verabreichung formuliert ist.

9. Verwendung nach Anspruch 8, wobei die Zusammensetzung den Opioidantagonisten in einer Menge von 0,1 Gew.-% bis 5 Gew.-% der Zusammensetzung umfasst.

10. Verwendung nach Anspruch 8, wobei die Zusammensetzung den Opioidantagonisten in einer Menge von 1 Gew.-% der Zusammensetzung umfasst.

11. Verwendung nach einem der Ansprüche 8 bis 10, wobei die Zusammensetzung in Form eines Gels, einer Creme, einer Salbe, einer Flüssigkeit, einer Suspension, einer Lösung, einer Emulsion, eines Schaums oder eines Aerosols vorliegt, der/die/das dem Individuum durch Auftragen oder Aufsprühen der Zusammensetzung auf die befallene Stelle verabreicht wird.

12. Verwendung nach einem der Ansprüche 8 bis 11, wobei der Opioidantagonist in einer Gesamttagesdosis von bis zu 150 mg/cm² Haut verabreicht wird.

13. Verwendung nach einem der Ansprüche 8 bis 11, wobei der Opioidantagonist in einer Gesamttagesdosis von bis zu 50 mg/cm² Haut verabreicht wird.

14. Verwendung nach Anspruch 7, wobei die Zusammensetzung für die orale Verabreichung formuliert ist.

15. Verwendung nach Anspruch 14, wobei die Zusammensetzung den Opioidantagonisten in einer Menge von 0,1 mg bis 10 mg umfasst.

16. Verwendung nach Anspruch 14, wobei die Zusammensetzung den Opioidantagonisten in einer Menge von 2 mg umfasst.

17. Verwendung nach einem der Ansprüche 14 bis 16, wobei die Zusammensetzung in Form einer Tablette oder Kapsel vorliegt.

18. Verwendung nach Anspruch 7, wobei die Zusammensetzung für die ophthalmische Verabreichung formuliert ist.

19. Verwendung nach Anspruch 18, wobei die Zusammensetzung den Opioidantagonisten in einer Menge von 0,1 Gew.-% bis 10 Gew.-% der Zusammensetzung umfasst.

20. Verwendung nach Anspruch 18 oder 19, wobei die Zusammensetzung in Form einer Lösung oder Suspension vorliegt.

21. Verwendung nach einem der Ansprüche 1 bis 20, wobei der Opioidantagonist ein- bis fünfmal pro Tag verabreicht wird.

22. Verwendung nach einem der Ansprüche 1 bis 21, wobei die Zusammensetzung weiterhin ein oder mehrere zusätzliche Agentien, die für die Behandlung des Leidens wirksam sind, umfasst.

23. Verwendung nach Anspruch 22, wobei es sich bei dem zusätzlichen Agens um Acyclovir, Valacyclovir oder Famciclovir handelt.

## Revendications

1. Utilisation d'un antagoniste d'opioïde ou un sel pharmaceutiquement acceptable de celui-ci dans la fabrication d'un médicament pour le traitement d'une affection causée par le virus varicelle-zona.

2. Utilisation selon la revendication 1, dans laquelle l'antagoniste d'opioïde est la naloxone.

3. Utilisation selon la revendication 1, dans laquelle l'antagoniste d'opioïde est le nalméfène.

4. Utilisation selon la revendication 1, dans laquelle l'antagoniste d'opioïde est la naltrexone.

5. Utilisation selon l'une quelconque des revendications 1 à 4, dans laquelle l'affection causée par le virus varicelle-zona est le zona.

6. Utilisation selon l'une quelconque des revendications 1 à 4, dans laquelle l'affection causée par le virus varicelle-zona est le zona ophtalmique.

7. Utilisation selon l'une quelconque des revendications 1 à 6, dans laquelle l'antagoniste d'opioïde est administré sous la forme d'une composition comprenant l'antagoniste d'opioïde et au moins un excipient pharmaceutiquement acceptable.

8. Utilisation selon la revendication 7, dans laquelle la composition est formulée pour administration topique.

9. Utilisation selon la revendication 8, dans laquelle la composition comprend l'antagoniste d'opioïde en une quantité de 0,1 % à 5 % en poids de la composition.

10. Utilisation selon la revendication 8, dans laquelle la composition comprend l'antagoniste d'opioïde en une quantité de 1 % en poids de la composition.

11. Utilisation selon l'une quelconque des revendications 8 à 10, dans laquelle la composition est sous la forme d'un gel, une crème, une pommade, un liquide, une suspension, une solution, une émulsion, une mousse ou un aérosol administré au sujet par étalement ou pulvérisation de la composition sur la zone affectée.

12. Utilisation selon l'une quelconque des revendications 8 à 11, dans laquelle l'antagoniste d'opioïde est administré à une dose journalière totale allant jusqu'à 150 mg/cm² de peau.

13. Utilisation selon l'une quelconque des revendications 8 à 11, dans laquelle l'antagoniste d'opioïde est administré à une dose journalière totale de 50 mg/cm² de peau.

14. Utilisation selon la revendication 7, dans laquelle la composition est formulée pour administration orale.

15. Utilisation selon la revendication 14, dans laquelle la composition comprend l'antagoniste d'opioïde en une quantité de 0,1 mg à 10 mg.

16. Utilisation selon la revendication 14, dans laquelle la composition comprend l'antagoniste d'opioïde en une quantité de 2 mg.

17. Utilisation selon l'une quelconque des revendications 14 à 16, dans laquelle la composition est sous la forme d'un comprimé ou une capsule.

18. Utilisation selon la revendication 7, dans laquelle la composition est formulée pour administration ophtalmique.

19. Utilisation selon la revendication 18, dans laquelle la composition comprend l'antagoniste d'opioïde en une quantité de 0,1 % à 10 % en poids de la composition.

20. Utilisation selon la revendication 18 ou 19, dans laquelle la composition est sous la forme d'une solution ou une suspension.

21. Utilisation selon l'une quelconque des revendications 1 à 20, dans laquelle l'antagoniste d'opioïde est administré 1 à 5 fois par jour.

22. Utilisation selon l'une quelconque des revendications 1 à 21, dans laquelle la composition comprend en outre un ou plusieurs agents additionnels efficaces pour traiter l'affection.

23. Utilisation selon la revendication 22, dans laquelle l'agent additionnel est l'acyclovir, le valacyclovir, ou le famciclovir.
